Europäisches Patentamt

European Patent Office

Office européen des brevets

(11) Publication number: **0 235 453**
A1

(12) **EUROPEAN PATENT APPLICATION**

(21) Application number: 86309458.7

(22) Date of filing: 04.12.86

(51) Int. Cl.⁴: **A 61 B 17/36,** A 61 F 9/00

(30) Priority: **12.12.85 US 808001**

(43) Date of publication of application: **09.09.87**
**Bulletin 87/37**

(84) Designated Contracting States: **DE FR GB**

(71) Applicant: **BAXTER TRAVENOL LABORATORIES, INC.,**
**One Baxter Parkway, Deerfield, IL 60015 (US)**

(72) Inventor: **Chou, Jim-Son, 3756 Bagley Avenue 206, Los**
**Angeles CA 90034 (US)**

(74) Representative: **Day, Jeremy John et al, REDDIE &**
**GROSE 16 Theobalds Road, London, WC1X 8PL (GB)**

(54) **Surgical laser instrument.**

(57) A surgical laser instrument for making at least one slit in the cornea (15) comprising a surgical laser for generating a surgical laser beam, laser beam delivery optics (11) for delivering the surgical laser beam to the cornea (15) and a microscope for facilitating viewing of the cornea (15). The laser beam delivery optics (11) focuses the surgical laser beam to a narrow focal plane (31) substantially at the cornea (15). The focal plane (31) is substantially tangent to the cornea (15) at a location intermediate the ends of the focal plane (31) so that the surgical laser beam can make a slit in the cornea (15). This tangency relationship between the focal plane (31) and the cornea (15) delivers more even laser energy to the cornea (15) along the full length of the focal plane (31) so that the depth of the slit is more nearly constant.

D-1803

## SURGICAL LASER INSTRUMENT

## BACKGROUND OF THE INVENTION

Radial keratotomy is a surgical procedure in which radial incisions are made in the cornea in order to change its shape and move the vision focal point onto the retina. This procedure is used to treat myopia.

Currently, this procedure is performed with a diamond knife, and the formation of the slits with a knife can produce trauma. The incisions are typically made to approximately 90 percent of the depth of the cornea. Because the cornea is only about .5mm thick, each of the incisions must be made with great care and precision. As would be expected for surgery of this kind, there are various sources of potential error.

Trokel et al have reported (Excimer Laser Surgery of The Cornea, American Journal Of Ophthamology, 96:710-715, 1983), in vitro studies in which an excimer laser was used to remove corneal tissue through photochemical laser-tissue interaction. The authors state that this interaction is not thermal and does not involve optical breakdown, but rather it directly breaks organic molecular bonds without heating the tissue.

Cornea surgery with a laser, being a noncontact procedure, could be less traumatic than the diamond knife procedure described above. However, there a number of problems in utilizing a laser for corneal tissue removal.

For example, a slit formed in the cornea by a laser during radial keratomomy should be of essentially constant depth. However, because the cornea is curved and the focal plane of the laser beam is straight, laser energy received by the cornea would not be equal along the full length of the focal plane, and consequently, this would lead to some variation in depth of the slit formed by the laser.

Because radial keratomomy typically requires several radial slits, the laser surgical instrument should be capable of providing several of the slits at precisely controlled locations in the cornea. I am not aware of any laser surgical instrument which will do this.

The preferred surgical laser operates in the ultraviolet spectrum, and for this reason, it is preferred to provide an aiming laser having an aiming laser beam in the visible spectrum to enable the surgeon to observe the location of the surgical laser beam. In a conventional slit lamp, an optical train is used to deliver both laser beams along a common optical path, and the refracting elements of the optical train cause the focal planes of the two laser beams to be at slightly different locations. It is known to make corrections for this with a technique known as color compensation. However, color compensation is very difficult or impossible to use for lasers operating in the far ultraviolet portion of the spectrum in combination with a visible laser beam.

## SUMMARY OF THE INVENTION

This invention provides a laser surgical instrument which solves these problems. For example, this invention enables a surgical laser beam to make slits in curved tissue surfaces which are of more even depth throughout. The instrument is particularly adapted for radial keratomomy in that it enables the formation of a plurality of radially extending slits arranged in a generally annular configuration. Finally, the instrument causes the focal planes of the surgical and aiming laser beams to be substantially coincident even when utilizing a surgical laser operating in the far ultraviolet portion of the spectrum, and no color compensation is required.

To provide a slit in the cornea of more even depth, this invention orients the focal plane of the surgical laser beam and the cornea so that the focal plane initially impinges on the cornea at least at a location intermediate the ends of the focal plane. With this arrangement, the focal plane is closely adjacent the cornea along its full length and, therefore, the laser energy absorbed by the cornea, and hence the depth of the slit, is more likely to be more uniform. In the presently preferred system, the focal plane is essentially linear and is tangent to the cornea at a location intermediate the ends of the focal plane; however, a curved or other nonlinear focal plane may be used, if desired. To maximize uniformity of depth of the slit, said location is preferably about midway between the ends of the focal plane. Although this feature is particularly adapted for making radial incisions in the cornea, it can be utilized to make incisions having any desired orientation in the cornea or for making incisions in other curved body regions.

The surgical laser beam has generally parallel longitudinal edges adjacent the focal plane, and the focal plane forms an acute angle with at least one of the longitudinal edges of the focal plane. With this arrangement, the focal plane, which forms an ablating edge, is properly angled to contact the cornea intermediate the ends of the focal plane. For corneal tissue removal, the acute angle is preferably from about 45 to about 65 degrees with 60 degrees being currently used. Although the acute angle can be formed in different ways, preferably, it is provided by focusing of the surgical laser beam using an appropriately angled mirror.

To accomplish this, laser beam delivery means is employed for delivering the surgical laser beam to the cornea. The laser beam delivery means includes means for focusing the surgical laser beam to a narrow focal plane.

In order to provide a plurality of radial slits arranged in a generally annular configuration, the surgical laser beam is rotated about a rotational axis which is spaced radially from the central axis of the surgical laser beam. This feature is particularly adapted for a radial keratomomy procedure, although its use is not necessarily so limited.

The preferred surgical laser operates in the far ultraviolet portion of the spectrum at 193 mm. This laser is preferred because the short wavelength lasers cannot penetrate deeply into tissue and lasers with shorter wavelengths experience too large an energy loss in traveling through ambient air. However, because the surgical laser beam from this laser is not visible, a light source in the form of an aiming laser which provides an aiming laser beam in the visible spectrum is preferably also employed.

The two laser beams are directed along a common optical path. To assure that the focal planes of the two laser beams will coincide, the laser beams are not refracted along such common optical path. Rather, the common optical path utilizes mirrors and no refracting elements to appropriately direct and focus the combined laser beams. By so doing, color compensation is eliminated.

In a preferred implementation, the laser surgical instrument includes a support and a microscope or other optical means mounted on the support to facilitate viewing of the cornea or other surgical field. At least a portion of the means for delivering the laser beam to the cornea is carried by the support, and such portion may include a housing mounted for rotation about a rotational axis relative to the support. The housing carries the laser beam focusing means, and the focusing means directs the surgical laser beam out of the housing, with the axis of the surgical laser beam being radially spaced from the rotational axis of the housing. This construction enables multiple slits arranged in an annular pattern to be formed in the cornea. In addition, the width of the focal plane and the slit formed thereby can be adjusted, and the means for doing this can be carried by the rotatable housing.

The invention, together with additional features and advantages thereof, may best be understood by reference to the following description taken in connection with the accompanying illustrative drawing.

BRIEF DESCRIPTION OF THE DRAWING

Fig. 1 is a side elevational view of a region of the human eye and a portion of the optical train for creating a slit in the cornea.

Fig. 1a is an enlargement of a region of Fig. 1.

Fig. 2 is a top plan view of the system of Fig. 1.

Fig. 3 is a front elevational view of a portion of a human eye following radial keratomomy.

Fig. 4 is a side elevational view of a laser surgical instrument embodying the optical train of Figs. 1 and 2.

Fig. 5 is a longitudinal sectional view of a region of Fig. 4 illustrating one preferred construction for the optical train of Figs. 1 and 2 and associated related structure.

Fig. 6 is a top plan view taken from the top of Fig. 5.

Figs. 7 and 8 are sectional views taken generally along lines 7-7 and 8-8, respectively, of Fig. 5.

DESCRIPTION OF THE PREFERRED EMBODIMENT

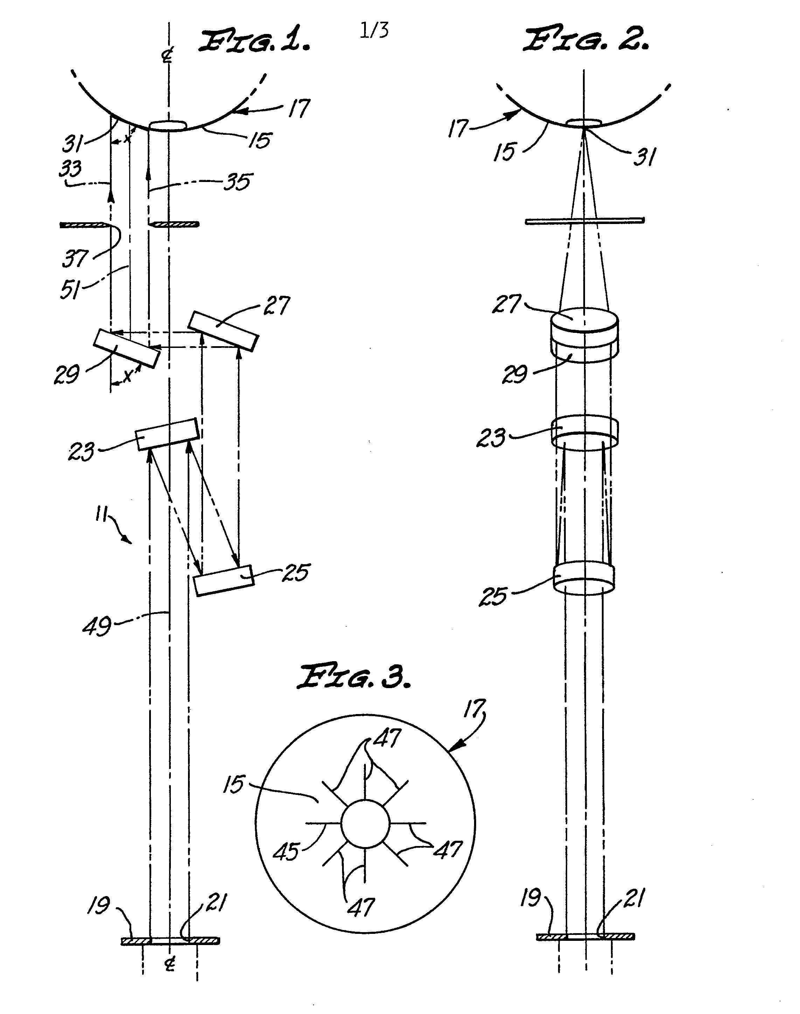

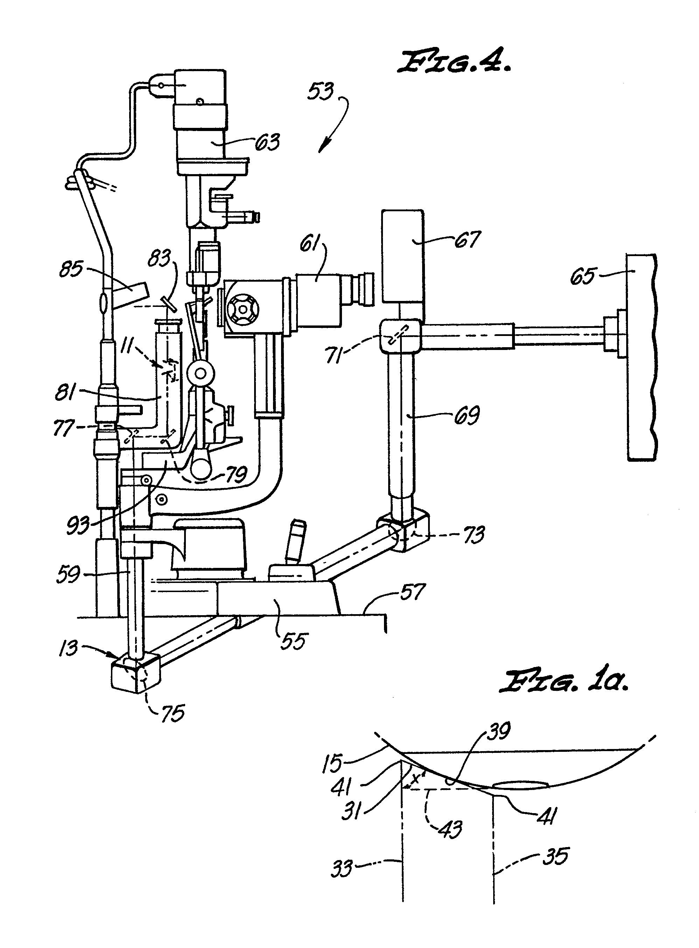

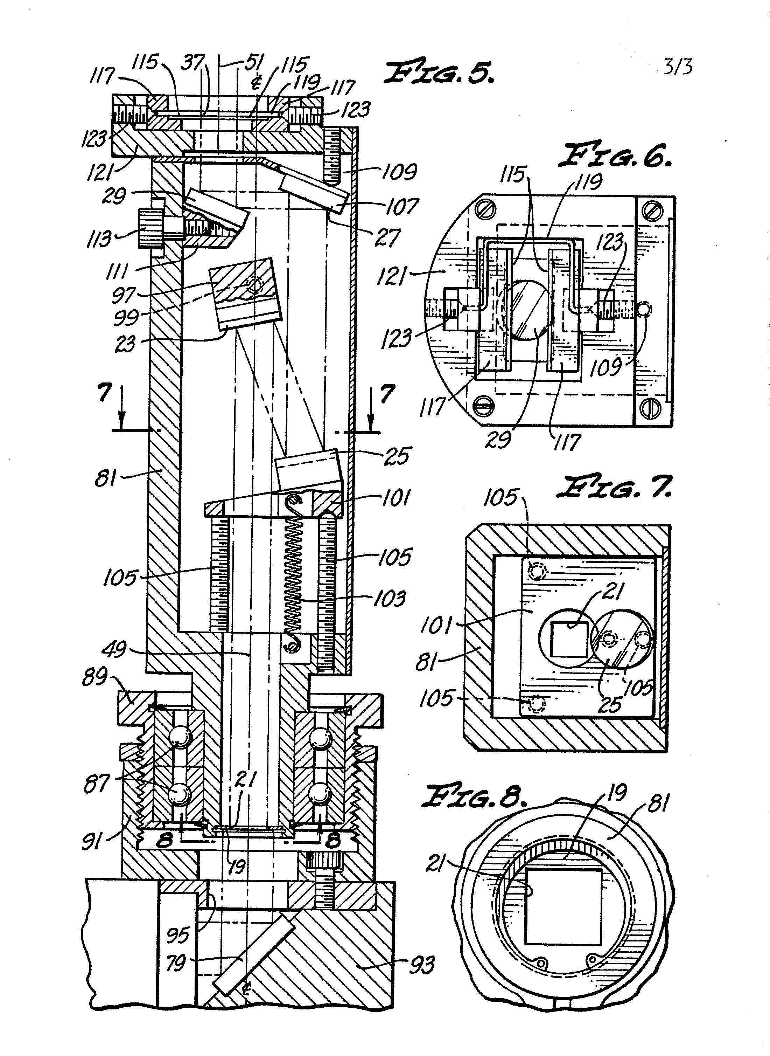

Figs. 1 and 2 show an optical system 11 of laser beam delivery means 13 (Fig. 4) for delivering a surgical laser beam and a visible aiming laser beam to a surgical field which, in this embodiment, is the cornea 15 of a human eye 17. The optical system 11 includes an optional mask 19 having a square aperture 21 which may be, for example, 6mm square, a convex mirror 23 for expanding the laser beam to, for example, 6mm by 12mm, a concave collimating mirror 25 for collimating the laser beam and directing it toward a flat mirror 27 and a concave focusing mirror 29 for focusing the laser beam to a narrow focal plane 31. The width of the laser beam as viewed in Fig. 1 remains constant. As shown in Fig. 1, the laser beam has parallel longitudinal edges 33 and 35, and the focal plane 31 forms an acute angle "X" with the edge 33. Although the angle "X" may vary, in this embodiment, it is 60 degrees. The length of the focal plane 31 as viewed in Fig. 1 can be adjusted or narrowed by reducing the size of an aperture 37 between the focusing mirror 29 and the cornea 15.

The focal plane 31 in the embodiment illustrated has a length of approximately 6 millimeters as shown in Fig. 1 and is extremely narrow and may have an extremely small width dimension such as 10 to 15 microns so that the focal plane may essentially be a focal line. Accordingly, the focal plane 31 forms, in effect, an ablating edge for the surgical laser beam.

As best seen in Fig. 1a, the focal plane 31 initially impinges on the cornea at least at a location intermediate the ends of the focal plane and, more specifically, is tangent to the cornea 15 at a point of tangency 39 which is intermediate the opposite ends 41 of the focal plane 31. Preferably, the point of tangency 39 is the midpoint between the ends 41. With this arrangement, the spacing between the focal plane 31 and the nearest location on the cornea 15 is minimized along the full length of the focal plane. This can be contrasted, for example, by utilizing a focal plane 43 which forms a 90 degree angle with the longitudinal edges 33 and 35 and is tangent to the cornea 15 at one end of the focal plane. As shown in Fig. 1a, the lefthand end of the focal plane 43 is spaced substantially from the cornea 15. Accordingly, the air gap between the lefthand edge of the focal plane 43 and the cornea 15 would attenuate the laser energy, particularly from a laser operating in the far ultraviolet portion of the spectrum, such that the regions of the slit formed by the lefthand edge of the focal plane 43 would not be expected to be as deep as at other regions of such slit.

The magnitude of the angle "X" is determined by the focusing mirror 29. Specifically, the angle between the reflective face of the mirror 29 and a vertical reference line is the angle "X" where the vertical reference line is parallel with the laser beam reflected by the mirror 29.

The laser beam at the focal plane 31 can form a radially extending slit or incision 45 in the cornea 15 as shown in Fig. 3. For example, the slit may be of the order of 6mm in length.

To form additional slits 47 in the cornea 15, which are identical to the slit 45, the optical system 11 can be rotated about a central rotational axis 49. As shown in Figs. 1 and 3, the laser beam reflected from the focusing mirror 29 has a central axis 51 which is spaced radially from the rotational axis 49. Consequently, the focal plane 31 is rotated in an annular pattern. By appropriately halting rotation and actuating a surgical laser, the slits 47 may be formed in sequence.

Figs. 4-8 illustrate one way in which the optical system 11 can be integrated into a surgical laser instrument 53. Except for the optical system 11 and related structure, the instrument 53 may be in the form of a conventional ophthalmic slit lamp, and for this reason, the basic structure of the instrument 53 is not described in detail herein.

The instrument 53 includes a support 55 (Fig. 4) which may include, or rest upon, a table 57. The instrument 53 has a vertical column 59 mounted on the support 55, and a microscope 61 or other suitable means for facilitating viewing of the surgical field is mounted on the column 59 for pivotal movement about the vertical axis of the column in a conventional manner. The eye upon which surgery is to be performed can be illuminated in a known manner by an illuminator 63 which also forms a part of the instrument 53.

A surgical laser 65 provides a surgical laser beam, and an aiming laser 67 provides a visible aiming laser beam. Although the surgical laser 65 could be of various different types, in this embodiment, it is an argon fluoride excimer laser which provides radiation at 193mm wavelength and which ablates tissue to form a slit or incision. Incisions with this laser should provide minimum thermal damage in adjacent unirradiated corneal tissue. Although the aiming laser 67 could also be of various different constructions, in this embodiment, it is a helium neon laser which produces radiation having a wavelength of 632mm.

The surgical and aiming laser beams are directed to the cornea 15 by the laser beam delivery means 13. Although the laser beam delivery means 13 can take different forms, in the embodiment illustrated, it includes an articulated arm 69 which may be of conventional construction and which has mounted therein flat mirrors 71, 73 and 75 for directing the laser beams from the laser 65 and 67 along a common optical path through the articulated arm and through the column 59. The laser beam delivery means 13 also includes flat mirrors 77 and 79 for directing the laser beams from the column 59 along a common optical path into a housing 81 which contains and supports the optical system 11. The laser beam delivery means includes the optical system 11 and a beam splitter 83 which directs a high percent of the surgical laser beam to the left as viewed in Fig. 4 against the cornea, and in this embodiment, a smaller percent of the visible aiming laser beam to the cornea for observation by the surgeon. The instrument 53 also includes a head support 85 for supporting the patient's head in the correct position so that the surgical laser beam can impinge on the cornea.

Figs. 5-8 show one way in which the optical system 11 and the related components can be constructed. The housing 81 is mounted for rotation about the rotational axis 49 in any suitable manner, such as by ball bearings 87 (Fig. 5) which are suitably retained within a bearing housing 89 threaded into an internally threaded cup 91 which is attached to a housing section 93 for the mirror 79. The mask 19 is suitably mounted in the lower end of the housing 81, and the housing section 93, the cup 91 and any intermediate members provide appropriate openings 95 to permit the flat mirror 79 to direct the laser beams through the aperture 21 of the mask 19.

Although various mounting constructions are possible, the convex mirror 23 is carried by a mounting block 97 suitably pivotally attached to the housing 81 so that it can be pivoted about a pivot axis 99 and locked in position at the desired angle with respect to the rotational axis 49. In this embodiment, the axis 99 is perpendicular to, and intersects, the rotational axis 49, and the aperture 21 and the laser beams passing therethrough are coaxial with the rotational axis 49.

The collimating mirror 25 is mounted for pivotal movement with a mounting block 101 which is resiliently retained by a spring 103 against the upper ends of three pins 105 (Figs. 5 and 7). By adjusting one or more of the threaded pins 105, the angle of the collimating mirror 25 can be correspondingly adjusted.

The flat mirror 27 is carried by a bracket 107 attached to the housing 81, and some angle change for the mirror 27 can be obtained by tightening of a screw 109 threaded through the upper end of the housing and bearing against the bracket 107. The bracket 107 has sufficient resilience to allow some change in angle of the flat mirror 27 in response to a force applied to the bracket 107 by the screw 109.

The focusing mirror 29 is mounted on a mounting block 111 by a screw 113. By rotating the screw 113, the mirror 29 can be adjusted about the axis of the screw 113. Of course, the mounting constructions for the various mirrors are purely illustrative. The focusing mirror 29 directs the laser beams out of the housing 81, and the width of these beams and the corresponding length of the focal plane 31 can be reduced by reducing the size of the aperture 37. Although this can be accomplished in various different ways, in the illustrated embodiment, the aperture 37 is defined by a pair of blades 115 (Figs. 5 and 6) carried by slides 117 resiliently biased apart by a spring 119 and slidable in a guide 121. The position of the slides 117, and hence of the blades 115, relative to each other can be controlled by set screws 123. Thus, by moving the set screws 123 to advance the blades 115 toward each other, the widths of the laser beams are reduced, and by moving the blades 115 away from each other, the widths of the laser beams can be increased up to the width they had when reflected by the focusing mirror 29.

The operation of the instrument 53 is in accordance with the operation as described in connection with Figs. 1-3.

Of course, the surgeon can observe the surgery through the microscope 61 in essentially the same manner as with other ophthalmic slit lamp surgery. Also, rotation of the laser beams about the axis 49 is accomplished by rotation of the housing 81. A suitable clamping or locking device (not shown) can be employed to fix the housing 81 in any of a plurality of desired rotational positions about the rotational axis 49.

Although an exemplary embodiment of the invention has been shown and described, many changes, modifications and substitutions may be made by one having ordinary skill in the art without necessarily departing from the spirit and scope of this invention.

CLAIMS

1. A surgical laser instrument comprising:

a surgical laser for generating a surgical laser beam;

laser beam delivery means for delivering the surgical laser beam to a surgical field;

optical means for facilitating viewing of the surgical field; and

said laser beam delivery means including means for focusing the surgical laser beam to a narrow focal plane with the surgical laser beam having general parallel longitudinal edges adjacent the focal plane and with the focal plane forming an acute angle with at least one of the longitudinal edges of the focal plane, said focal plane being adapted to be at the surgical field.

2. An instrument as defined in claim 1 wherein said acute angle is from about 45 to about 65 degrees.

3. An instrument as defined in claim 1 or claim 2 wherein said surgical laser beam is out of the visible spectrum and said instrument includes an aiming laser for generating an aiming laser beam in the visible spectrum, said laser beam delivery means includes means for delivering said laser beams along a common optical path without refracting the laser beams along said common optical path.

4. An instrument as defined in any one of claims 1 to 3 wherein the surgical laser beam from the focusing means has a central axis and said instrument includes means for rotating the surgical laser beam from the focusing means about a rotational axis which is spaced radially from the central axis of the surgical laser beam.

5. An instrument as defined in any one of claims 1 to 4 wherein said focusing means includes a mirror for focusing the surgical laser beam to said focal plane.

6. An instrument as defined in any of claims 1 to 5 including a support, said optical means includes a microscope mounted on said support, at least a portion of said laser beam delivery means is carried by said support, said portion of said laser beam delivery means includes a housing and means for mounting said housing for rotation about a rotational axis relative to said support, and said focusing means is carried by said housing and directs said surgical laser beam out of said housing with the central axis of the surgical laser beam being radially spaced from said rotational axis as the surgical laser beams leave the housing.

7. An instrument as defined in claim 6 wherein said portion of said laser beam delivery means includes a mask for defining the cross section of the surgical laser beam at one location in the housing, beam expansion means carried by the housing for expanding the cross section of the surgical laser beam beyond said one location and means carried by said housing for directing the expanded surgical laser beam onto the focusing means.

8. An instrument as defined in any of claims 1 to 7 including means for supporting a patient's head at a desired location and said laser beam delivery means includes means for focusing the surgical laser beam to the narrow focal plane substantially at the cornea of the patient with the focal plane initially impinging on the cornea at least at a location intermediate the ends of the focal plane whereby the surgical laser beam can make a slit in the cornea.

9. An instrument as defined in claim 8 wherein said location is about midway between the ends of the focal plane.

10. An instrument as defined in any of claims 1 to 9 including means for adjusting the length of said focal plane.

11. A surgical laser instrument comprising:

a surgical laser for generating a surgical laser beam which is out of the visible spectrum;

an aiming laser for generating an aiming laser beam in the visible spectrum;

laser beam delivery means for delivering said laser beams to a surgical field together along a common optical path without refracting the laser beams along said common optical path; and

optical means for facilitating viewing of the surgical field.

0235453

1/3

FIG. 1.

FIG. 2.

FIG. 3.

FIG.4.

FIG. 1a.

FIG. 5.

FIG. 6.

FIG. 7.

FIG. 8.

0235453

3/3

0235453

## EUROPEAN SEARCH REPORT

Application number

| DOCUMENTS CONSIDERED TO BE RELEVANT | | EP 86309458.7 |
|---|---|---|

| Category | Citation of document with indication, where appropriate, of relevant passages | Relevant to claim | CLASSIFICATION OF THE APPLICATION (Int. Cl. 4) |
|---|---|---|---|
| A | US - A - 4 396 285 (P.S.PRESTA et al.) | 1 | A 61 B 17/36 |
| X | * Totality; especially fig. 2; column 2, lines 45-48; column 4, lines 33-43 * | 11 | A 61 F 9/00 |
| Y | DE - A1 - 2 809 007 (MESSERSCHMIDT BOLKOW) <br> * Totality; fig.; page 5 * | 11 | |
| A | US - A - 4 091 814 (T.TOGO) | 1 | |
| Y | * Totality; especially column 3, lines 12-20 * | 11 | |
| A | DE - A - 1 514 165 (SIEMENS) <br> * Fig. 4; page 16, last paragraph - page 17, paragraph 1 * | 1 | |
| A | US - A - 4 520 824 (J.R.SWANINGER et al.) <br> * Totality * | 1 | TECHNICAL FIELDS SEARCHED (Int. Cl. 4) <br><br> A 61 B 17/00 <br> A 61 F 9/00 |
| D,A | AMERICAN JOURNAL OF OPHTALMOLOGY, vol. 96, 1983, Ophtalmic Publishing Company, Chicago <br><br> TROKEL et al. "Excimer Laser Surgery of the Cornea" pages 710-715 | 1 | |
| A | US - A - 4 461 294 (N.A.BARON) <br> * Totality * | | |

The present search report has been drawn up for all claims

| Place of search | Date of completion of the search | Examiner |
|---|---|---|
| VIENNA | 12-05-1987 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82